(19) Europäisches Patentamt
European Patent Office
Office européen des brevets

(11) Publication number: **0 169 547 B1**

(12) **EUROPEAN PATENT SPECIFICATION**

(45) Date of publication of patent specification: **21.09.88**

(51) Int. Cl.[4]: **C 07 C 85/06**, C 07 D 295/02, C 07 D 295/08

(21) Application number: **85109224.7**

(22) Date of filing: **23.07.85**

(54) **Selective syntheses of substituted amines.**

(30) Priority: **25.07.84 US 634503**

(43) Date of publication of application:
**29.01.86 Bulletin 86/05**

(45) Publication of the grant of the patent:
**21.09.88 Bulletin 88/38**

(84) Designated Contracting States:
**AT BE DE FR GB NL SE**

(56) References cited:
**GB-A- 747 841**
**US-A-3 283 002**
**US-A-3 708 539**
**US-A-3 767 709**
**JOURNAL OF THE CHEMICAL SOCIETY CHEMICAL COMMUNICATIONS, 1981, pages 611,612; R. GRIGG et al.: "Transition metal-catalysed N-alkylation of amines by alcohols"**
**THE JOURNAL OF ORGANIC CHEMISTRY, vol. 50, no. 9, 3rd May 1985, pages 1365-1370, American Chemical Society, Washington, D.C., US; Y. TSUJI et al.: "Ruthenium complex catalyzed N-heterocyclization. Syntheses of N-substituted piperidines, morpholines, and piperazines from amines and 1,5-diols"**

(73) Proprietor: **AIR PRODUCTS AND CHEMICALS, INC.**
**Route no. 222**
**Trexlertown Pennsylvania 18087 (US)**

(72) Inventor: **Marsella, John Anthony**
**744 N. Broad Street**
**Allentown, PA 18104 (US)**

(74) Representative: **Dipl.-Ing. Schwabe, Dr. Dr. Sandmair, Dr. Marx**
**Stuntzstrasse 16 Postfach 86 02 45**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 169 547 B1**

Courier Press, Leamington Spa, England.

## Description

Technical Field

1. Field of the Invention

The present invention relates to catalytic reactions of alkanediols with secondary amines and is particularly concerned with the controlled selective production of reaction products therefrom predominating respectively (1) in alkanolamines or (2) alkylenediamines.

2. Prior Art

It is known to produce certain N-substituted alkanediamines and various alkanolamines from dichloroalkanes and alkylene oxides, respectively. These starting materials are expensive and/or extremely toxic. The toxic nature of some of the alkylene oxides is a special problem for small-scale users, since the unit costs of installing safe-guards and monitoring systems increase with decreasing production scale.

Previous prior art attempts at aminating alkanediols have been limited to high temperature reactions utilizing heterogeneous catalysts. The high temperatures required in the prior art methods led to high operating pressures and low selectivities.

A limited number of prior art disclosures describe the use of homogeneous catalysts, e.g. $RhH(PPh_3)_4$, for the reaction of monoalcohols with amines. (See, for example, Grigg, et al, *J.C.S. Chem. Comm.*, pp 611—612 [1981]).

European Patent Publication No. 034,480 describes in general the preparation of N-alkylamine or N,N-dialkylamine by reacting a primary or secondary amine with a primary or secondary alcohol in the presence of certain noble metal catalysts, such as the metal, salt or complex of the noble metal. The preferred example of catalyst is a rhodium hydride-triphenylphosphine complex. Although the disclosure is concerned largely with reactions involving monofunctional alcohols, there is also disclosed the reaction of a primary amine with a diol for the formation of heterocyclic ring compounds containing the amine N atom. For this purpose, the diol used should contain at least four atoms in the chain so that cyclization can occur. The publication contains no disclosure of reaction of a diol with secondary amine, wherein cyclization is not possible.

An article by Murahashi, et al. in *Tetrahedron Letters* (vol. 23, No. 2, pp. 229—232, [1982]) describes the synthesis of secondary amines by reaction of alcohols with amines in the presence of $RuH_2(PPh_3)_4$ catalyst. By the reaction of butane diol or higher alkane diols with n-hexylamine, N-heterocyclic compounds are formed.

U.S. Patent 3,708,539 discloses the condensation of amines with alcohols in the presence of ruthenium or certain other noble metal catalysts introduced as halides. The process is preferably conducted in the presence of a biphilic ligand of the structure $ER_3$, wherein E may be phosphorus or arsenic. Particular examples are directed to (1) reaction of butanol with dibutylamine obtaining tributylamine; (2) using hexanol as reactant in the same manner resulted in the formation of dibutylhexylamine.

Reaction of diols with ammonia or alkylamines to produce diaminoalkanes is disclosed in U.S. Patent 3,270,059. The reaction is carried out in the presence of hydrogen at 150—300°C and at a pressure of at least 10 atmospheres, over solid catalysts which contain at least one metal from the group consisting of cobalt and nickel. When secondary amine is employed as reactant, tertiary diamines are obtained. Reaction of ethylene glycol with diethylamine under the conditions of the patent yields chiefly tetraethylethylene diamine and a lesser amount of diethylethanolamine.

The selective conversion of primary amines into (I) N,N-dimethylalkyl- or (II) N,N-dialkylmethyl-amines by reaction with methanol in the presence of $RuCl_2(Ph_3P)_3$ catalyst, is disclosed in an article by Arcelli, et al. in the Journal of Organometallic Chemistry (vol. 235, pp. 93—96 [1982]). The selectivity towards the I or II type compound is controlled by choice of the amount of catalyst and the ratio of reactants.

Summary of the Invention

In accordance with the present invention, tertiary alkanolamines and alkanediamines are selectively produced in high yield from alkanediols by reaction with secondary amines in the presence of ruthenium or iridium complexes. Selectivity favoring production of alkanolamines (i.e., mono-amination) is achieved by the use of a complex of a ruthenium compound with a ligand such as triphenylphosphine or a mixture of the ruthenium compound with such ligand. Selectivity toward production of alkanediamines is achieved by the use of an iridium complex catalyst, or of the ruthenium compound in the absence of the phosphine component.

Detailed Description

In practice of the invention, a solution of a secondary amine and alkanediol is stirred in the presence of the ruthenium or iridium catalyst at 50—200°C for two to six hours. The preferred temperature range is at about 80 to 160°C, with lower temperatures giving lower reaction rates and higher temperatures tending to effect dehydrogenation and/or decarbonylation of the diol.

The secondary amines can be represented by the formula: $HNR_2$ in which

$-NR_2$ is 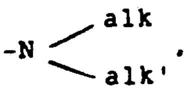, 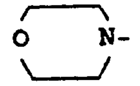, or 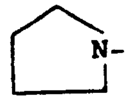.

and in which "alk" and "alk'" are alkyl groups of up to 20 carbon atoms.

The alkane diols can include up to 12 carbon atoms with a linear or branched carbon skeleton. Preferably the hydroxyl groups of the alcohol functionalities should be separated by not more than one carbon. Most preferably, the diols should be vicinal diols. Example of most preferred diols include ethylene glycol, 1,2-propanediol, 2,3-butanediol, 3-methyl-1,2-butanediol, 1,2-cyclohexanediol and 1,2-cyclododecanediol.

The concentration of the secondary amine may be in the range 0.5 to 10 mols per liter of reaction medium, preferably 1 to 5 mols/liter. The catalyst concentration may be generally in the range of $10^{-4}$ to $10^{-1}$ mols per liter of reaction medium, preferably $10^{-3}$ to $10^{-2}$ mols/liter.

Selectivity of the reaction can be altered to give mainly mono-amination (production of alkanolamines) or di-amination (production of alkylene diamines), by proper choice of catalyst. Proven catalysts that favor mono-amination are: $RuCl_2(PPh_3)_3$, $RuHCl(PPh_3)_3$; $RuCl_3.xH_2O$ admixed with about 3 moles/mole of $PPh_3$; $[Ru(NH_3)_6]Cl_3$ admixed with 3 moles/mole of $PPh_3$; $K_2[(RuCl_5)_2O]$, $Ru(NO)Cl_3$, $K_2RuCl_5$, $K_2RuCl_6$, and $[Ru(NH_3)_6]Cl_2$, each admixed with about 3 moles/mole of $PPh_3$. Also found to favor mono-amination was $K_2RuO_4$ admixed with about 5 moles/mole $PPh_3$. For these ruthenium-based systems, the selectivity preference progresses smoothly from mono- to di-amination as the amount of $PPh_3$ is decreased. In addition to the above listed ruthenium complexes other catalysts which are expected to favor mono-amination include: $K_2RuCl_6$, $K_2RuCl_5$, $RuCl_2(DMSO)_4$, "Ruthenium Red" (ammoniated ruthenium oxychloride), anhydrous $RuCl_3$, etc., each in admixture or molecular association with triphenylphosphine.

In general, based on observations, it can be stated that triarylphosphines substituted in the para position behave quite similarly to unsubstituted triphenylphosphine in admixture or in chemical combination with the listed ruthenium catalytic compounds. Those with ortho substituents show decreased rates of reaction with the selectivity tending toward diamination. The situation is more complex in the case of mixed aryl-alkylphosphines, including the potentially chelating diphosphines, bis(diphenylphosphino)methane (DPPM) and bis(diphenylphosphino)ethane (DPPE). Also included in this category are triphenylphosphite and tri-isopropylphosphine. However, in most cases, it was observed that mono-amination is favored over di-amination by the addition of organic phosphine ligand as compared to the catalytic reaction in the absence of phosphine. As indicated by the listed examples, the organic phosphine compound may be initially introduced into the reaction medium as a separate component or in a form chemically combined with the platinum group metal catalyst. The organic phosphine compound or complex preferably is one corresponding to the formula

$$PR_1R_2R_3$$

wherein $R_1$ and $R_2$ are each hydrogen or an alkyl or aryl hydrocarbyl group and $R_3$ is a hydrocarbyl group; each said hydrocarbyl group separately containing up to 12 carbon atoms.

Di-amination is selectively favored by $RuCl_3.xH_2O$ (phosphine-free), and by $IrCl_3.xH_2O$ (phosphine-free) or in admixture with triphenylphosphine, as well as by phosphine-free ruthenium mixtures and complexes such as: $K_2[(RuCl_5)_2O]$, $Ru(NO)Cl_3$, $K_2RuCl_5$, $K_2RuCl_6$, $RuCl_2(DMSO)_4$, and "Ruthenium Red", in the absence of mixed or chemically associated phosphines.

The catalysts employed in practice of the invention, without being bound to any particular theory, apparently function as homogeneous catalysts, since they are at least partially dissolved in the reaction medium. As a result, such catalysts obtain more selective product distribution than that obtained using heterogeneous catalysts. Moreover, catalyst modifiers, such as the triphenylphosphine in the instant case, have a marked effect on the activity of homogeneous catalysts. Thus, by the practice of the present invention, the selective manufacture of desired alkanolamines or of the desired di-amines is made possible utilizing readily available and relatively low toxicity starting materials. Furthermore, the desired products are readily obtained under relatively mild operating conditions, preferably at autogenous pressure, and temperatures in the range of 50 to 200°C, preferably at 110 to 160°C; without requiring addition of hydrogen to the system, although hydrogen may be employed, if desired.

The exact composition and structure of the active catalyst species promoting the reaction is not clear, since the form in which the catalyst is introduced may function merely as a precursor to the active structure formed in the medium under reaction conditions. While carbonyl complexes have been observed in reaction mixtures, the use of isolated neutral carbonyl complexes of ruthenium as such catalyst precursors were found to lead to lower catalytic activity.

Among other active catalysts favoring di-amination, good results are obtained with $IrH(Cl)_2(PPh_3)_3$. Iridium carbonyl complexes, like the ruthenium carbonyl complexes, show decreased overall activity. Surprisingly, however, $IrH_2Cl(PPh)_3$ showed relatively low overall activity, but favored di-amination.

The process of the invention may be carried out in the presence of added solvents or diluents, among which are preferred: N-methylpyrrolidinone, N,N-dimethylacetamide, dimethylsulfoxide (DMSO), water, 1,2-dimethoxyethane.

While on the basis of prior art disclosures, it was expected that ruthenium and rhodium-based catalysts would show similar behavior in the promotion of amination reactions, it was unexpectedly found in preliminary experiments that this was not the case in reactions of diols with secondary amines.

A number of exploratory runs were made including (a) reaction of pyrrolidine with methanol over the prior art $RhH(PPh_3)_4$ catalyst; (b) reaction of pyrrolidine with ethylene glycol over the same rhodium complex and (c) over $RuCl_2(PPh_3)_3$. These runs and all of the runs hereinafter reported were carried out under nitrogen atmosphere. The GC analyses were performed using a copper column with 15% Carbowax 20M on Gaschrom Q as the stationary phase. All reaction vessels were charged in a glove box under an inert ($N_2$) atmosphere.

Example A

A solution of 6.0 ml pyrrolidine (5.1 g) in 50.0 ml methanol was prepared. A small flask equipped with a septum sealed side arm was charged with 0.27 g $RhH(PPh_3)_4$ ($2.3 \times 10^{-4}$ mol) and fitted with a reflux condenser. To this flask was added 3.5 ml (*ca* $5 \times 10^{-3}$ mol pyrrolidine) of the above solution. The mixture was refluxed by placing in a previously heated oil bath.

The reaction was monitored periodically by GC, with N-methylpyrrolidine being observed. After five hours the reaction was stopped. At no time was a homogeneous solution obtained. GC analysis revealed 45% conversion with 96% selectivity to the formation of N-methylpyrrolidine.

The foregoing run was made in accordance with the work reported by Grigg, et al. in the *J.C.S. Chem. Comm.* cited above, in which $RhH(PPh_3)_4$ is stated to be the most active catalyst among the several transition metal phosphine complexes used in the amination of alcohols. It was confirmed by the run made in this example (A) that the rhodium catalyst employed does show some activity for amination at low temperature (refluxing methanol ~65°C).

Example B

This run was carried out to test the activity of $RhH(PPh_3)_4$ as a catalyst for amination of ethylene glycol.

A 22 ml Parr vessel was charged with 0.15 g $RhH(PPh_3)_4$ ($1.3 \times 10^{-4}$ mol), 5.0 ml ethylene glycol and 1.0 ml pyrrolidine (0.85 g, $1.2 \times 10^{-2}$ mol).

The vessel was placed in a hot oil bath (180°C) and the contents were stirred magnetically for six hours. During this time the pressure rose to 75 psig. Upon cooling to room temperature, the pressure fell to 21 psig. The reactor was opened to reveal a deep red solution and black solid. GC analysis showed a large number of low retention time peaks, one of which had the same retention time as pyrrolidine. Smaller peaks were also seen corresponding to retention times of N-(β-hydroxyethyl)pyrrolidine and 1,2 bis(pyrrolidino)ethane. The latter was observed as a shoulder on the large ethylene glycol peak. Unidentified higher boiling compounds were also present.

The foregoing experiments (Example B) indicate that these rhodium complexes have some activity at higher temperature (≥ 150°C) in the promotion of the amination reaction, giving a mixture of products, including minor amounts of N-(β-hydroxyethyl)pyrrolidine and 1,2 bis(pyrrolidino)ethane. The difference in behaviour of the catalyst in amination of glycol as compared to its observed activity in amination of methanol could not be satisfactorily explained, although various theories could be advanced based on considerations of likely mechanisms for amination of alcohols.

Example C

Under the same conditions (120—125°C) reported in operating Example 1 (below) a run was carried out using as catalyst $RhCl_3 \cdot 3H_2O$ (43% Rh) admixed with 3 moles $PPh_3$ per mole Rh, in the amination of ethylene glycol by reaction with morpholine. The overall conversion to aminated products was 27% with 23% selectivity in the production of hydroxyethylmorpholine and 42% selectivity in the production of bis(morpholino)ethane.

Example D

Under conditions substantially similar to Example B, but using $RuCl_2(PPh_3)_3$ at that temperature (180°C), large amounts of N-(β-hydroxyethyl)pyrrolidine and smaller amounts of 1,2 bis(pyrrolidino)ethane were obtained from the reaction of ethylene glycol with pyrrolidine.

Following the procedure of Example B, 0.12 g $RuCl_2(PPh_3)_3$ ($1.3 \times 10^{-4}$ mol) was employed in lieu of the rhodium catalyst. In this case, the reactor pressure at room temperature was 63 psig at the end of the reaction. The reaction mixture was a dark yellow color with a black solid also evident. GC analysis showed a large amount (60% yield) of N-(β-hydroxyethyl)pyrrolidine and some 1,2-bis pyrrolidino-ethane to be present. This was confirmed by GC—MS, which also showed small amounts of N-methyl- and N-ethylpyrrolidine to be present in the reaction products. No pyrrolidine was observed, although some benzene was present. In a separate run, the gases were collected and analyzed; hydrogen was found to be a major component.

Subsequent experiments using the ruthenium complex catalyst showed the advantages obtained when using lower reaction temperature in the range of 100—125°C. These lower temperature runs, reported in Tables 1 to 4 below and as indicated in Table 5, were made with the indicated catalysts using the procedures outlined above. In these runs the reactions at 120°C were carried out in a 22 ml Parr stainless steel pressure vessel while the reactions at 100° and 110°C with liquid sampling using the glassware described in Example A. Quantitation was by the internal standard method with 1-methyl-2-pyrrolidinone added as the reference. Liquid amines were added directly to the reaction vessel while gaseous amines were first dissolved in ethylene glycol or other diol to form a solution of known concentration, of which a known volume was charged to the reaction vessel.

Operating Examples

Example 1

A series of experimental runs were carried out with various diols and different secondary amines at a reaction temperature of 120—125°C for 2—2.5 hours. The amine concentration in each of the runs was about 1.8 M and the catalyst concentration about $2 \times 10^{-2}$M. The reactants and catalysts employed in these runs are set out in Table 1, wherein the reactants are designated by: EG=ethylene glycol, PRDIOL=propanediol, MOR=morpholine, DMA=dimethylamine, DEA=diethylamine, DIPA=diisopropylamine. Total selectivity (selec) is defined as

$$\frac{Ym + Yd}{Conversion} \times 100\%,$$ where Ym = yield of mono-aminated product(s) and Yd = yield of diaminated product; $r = \frac{Yd}{Ym + Yd}$

Table 1

| Run No. | Amine | Diol | Catalyst | Conversion % | Total Selec % | r |
|---|---|---|---|---|---|---|
| 1 | MOR | EG | $RuCl_2(PPh_3)_3$ | 100 | 92 | 0.09 |
| 2 | DMA | EG | $RuCl_2(PPh_3)_3$ | 100 | 85 | 0.04 |
| 3 | DMA | 1,2 PRDIOL | $RuCl_2(PPh_3)_3$ | 67 | 95 | 0.06 |
| 4 | DMA | 1,3 PRDIOL | $RuCl_2(PPh_3)_3$ | 85 | 66 | 0.01 |
| 5 | DIPA | EG | $RuCl_2(PPh_3)_3$ | 20 | 100 | 0 |
| 6 | MOR | EG | $RuCl_3 \cdot xH_2O$ | 100 | 96 | 0.83 |
| 7 | DMA | EG | $RuCl_3 \cdot xH_2O$ | ca 90 | >90 | 0.89 |
| 8 | DMA | 1,2-PRDIOL | $RuCl_3 \cdot xH_2O$ | 41 | 81 | 0.39 |
| 9 | DMA | 1,3-PRDIOL | $RuCl_3 \cdot xH_2O$ | 70 | 51 | 0.88 |
| 10 | MOR | EG | $IrCl_3 + 3PPh_3$ | 79 | 90 | 0.89 |
| 11 | DMA | EG | $IrCl_3 + 3PPh_3$ | 76 | 72 | 0.87 |
| 12 | DMA | 1,2-PRDIOL | $IrCl_3 + 3PPh_3$ | 36 | 66 | 0.36 |
| 13 | DMA | 1,3-PRDIOL | $IrCl_3 + 3PPh_3$ | 45 | 43 | 0.93 |
| 14 | DEA | EG | $RuCl_2(PPh_3)_2$ | 98 | 92 | 0.01 |
| 15 | DEA | EG | $RuCl_3 \cdot xH_2O$ | 42 | 98 | 0.85 |

Example 2

Another series of runs was carried out under conditions of Example 1 to determine the effect of the triphenylphosphine to ruthenium ratio (P:Ru) on the reaction of ethylene glycol with morpholine, catalyzed by mixtures of $RuCl_3.xH_2O$ with $PPh_3$. The results are reported in Table 2.

Table 2

| Run | P:Ru | Conversion % | r | Total Selectivity % |
|---|---|---|---|---|
| 14 | 0 | 60 | .91 | 82 |
| 15 | 0 | 100 | .83 | 95 |
| 16 | 0 | 100 | .83 | 96 |
| 17 | 0.5 | 53 | .53 | 91 |
| 18 | 1.0 | 79 | .22 | 90 |
| 19 | 3.0 | 95 | .05 | 91 |

As seen from the above-tabulated results, by the judicious use of triphenylphosphine as catalyst modifier with ruthenium complexes, selectivity of the reaction can be altered to obtain high conversion to (i) mono-aminated or (ii) di-aminated products, as represented by the equations:

$$HOCH_2CH_2OH + HNR_2 \rightarrow HOCH_2CH_2NR_2 + H_2O \quad (i)$$

$$HOCH_2CH_2OH + 2HNR_2 \rightarrow R_2NCH_2CH_2NR_2 + 2H_2O \quad (ii)$$

in which $-NR_2$ is the same as that defined under the Detailed Description.

Example 3

Another series of runs was carried out in the amination of ethylene glycol with various secondary amines in the presence of $RuCl_2(PPh_3)_3$ as the added catalyst at 120°C. These runs were made using 5 ml of glycol to 0.011—0.012 mol. amine and 1 mol% Ru (based on amine). The results are tabulated in Table 3.

Table 3

| Amine | pyrrolidine | morpholine | dimethylamine |
|---|---|---|---|
| Time (hr) | 6 | 2 | 3 |
| Conv. (%) | 100 | 100 | 100 |
| Selectivity (%) | | | |
| $R_2NCH_2CH_2OH$ | 79 | 83 | 81 |
| $R_2NCH_2CH_2NR_2$ | (observed) | 9 | 4 |

It will be seen from Table 3 that high conversion is obtained with the ruthenium catalyst at moderate temperature, and unexpectedly high selectivity to substituted ethanolamines.

Some increase in pressure was noted in the runs at 120°C, but these increases amounted to only about 15 psig (at 25°C) in a 22 ml Parr vessel. In contrast, higher temperature runs led to net pressure increases of 60—70 psig. Thus, the lower temperature apparently prevents hydrogen loss from the reaction system — an observation consistent with higher selectivity to the simple amination products at the lower temperatures.

The effect of varying the starting Ru complex on product distributions is shown in Table 4. These runs were made using ethylene glycol with morpholine as the secondary amine; 5 ml of glycol were used per gram of morpholine (=.0115 mol) and 1 mol% Ru (based on morpholine).

Table 4

| Complex | $RuCl_2 \cdot (PPh_3)_3$ | $RuHCL \cdot (PPh_3)_3$ | $RuCl_3 \cdot xH_2O + 3PPh_3$ | $RuCl_3 \cdot xH_2O$ |
|---|---|---|---|---|
| Time (hr) | 6 | 5.5 | 2 | 2 |
| Temp (°C) | 100 | 100 | 120 | 120 |
| Conv (%) | 89 | 94 | 95 | 100 |
| Selec (%): | | | | |
| $R_2NCH_2CH_2OH$ | 94 | 90 | 88 | 15 |
| $R_2NCH_2CH_2NR_2$ | 3 | 2 | 2 | 80 |

Example 4

While in the previous examples the secondary amine employed was free of other functional groups, the invention is also applicable to functionally substituted secondary amines.

The procedure described in Example 3 was employed using N,N,N'-trimethylethylenediamine as the secondary amine. The reaction product was analyzed by gas-liquid chromatography and was found that 2-[[2-(dimethylamino)ethyl]methylamino]ethanol had been formed in 91% yield, in a 2.5 hour operation.

Example 5

While the reactions in accordance with the invention do not require the presence of added hydrogen, it may be desired in some instances to carry out the process in the presence of hydrogen.

The procedure described in Example 3 was employed in the amination of ethylene glycol with morpholine as the secondary amine. The reaction vessel was charged with hydrogen (at 25°C) to a pressure of 50 psig (=4.55 Kg/cm$^2$). After heating the contents of the reactor at 125°C for 2.5 hours, the vessel was cooled and vented. Analysis by gas-liquid chromatography showed 60% conversion of the morpholine to N-2-(hydroxyethyl)morpholine (63% selectivity) and 1,2-bis(morpholino)ethane (30% selectivity). From the foregoing run it will be seen that the presence of hydrogen leads to a retardation of the reaction rate as well as to alteration of reaction selectivity. Thus, the ratio in this instance was r=0.32, as compared to r=0.09 in Run No. 1 of Table 1.

A number of runs were carried out to determine the effect of various phosphine additives on $RuCl_3.xH_2O$ catalyzed reactions of morpholine with ethylene glycol. The conditions employed were substantially the same as used in Example 1, unless otherwise indicated. The results are reported in Table 5.

Table 5

| L | L:Ru | Temp (°C) | Time hrs. | Conv. (%) | Total Selec(%) | r |
|---|---|---|---|---|---|---|
| $P(p-C_6H_4F)_3$ | 3.0 | 120 | 2.5 | 100 | 95 | <0.01 |
| $P(Ph)Me_2$ | 3.0 | 125 | 2.5 | 100 | 88 | 0.08 |
| $P(p-tol)_3$ | 2.9 | 128 | 2.25 | 100 | 93 | 0.17 |
| $P(C_6F_5)_3$ | 2.5 | 120 | 3 | 22 | 60 | >0.6 |
| $P(OPh)_3$ | 3.2 | 125 | 2.5 | 13 | 48 | 0.69 |
| DPPM | 1 | 120 | 2 | 70 | 91 | 0.73 |
| $PPh_2Me$ | 3.0 | 125 | 2.5 | 45 | 88 | 0.73 |
| $P(i-Pr)_3$ | 3.8 | 120 | 2.5 | 12 | 44 | 0.75 |
| DPPE | 1 | 125 | 2 | 13 | 57 | 0.82 |
| $PPh(C_6F_5)_2$ | 3.6 | 117 | 3 | 100 | 84 | 0.84 |
| $P(o-C_6H_4NMe_2)$ | 2.7 | 120 | 2 | 37 | 90 | 0.94 |
| $P(o-tol)_3$ | 2.9 | 130 | 2.5 | 48 | 85 | 0.95 |

Me=methyl; Ph=phenyl; tol=tolyl;
DPPM=bis (diphenylphosphino)methane
DPPE=bis (diphenylphosphino)ethane.

For the purposes of this disclosure, ruthenium based catalyst systems are considered to be di-amination catalysts if they give values of r for a given reaction comparable to or larger than the value of r obtained for that reaction using $RuCl_3.xH_2O$ alone.

Other runs made under the reported conditions gave the results shown in Table 6.

Table 6

| Catalyst | % Conv. | r | % Total Selectivity |
|---|---|---|---|
| $[(MePh_2P)_3Ru(\mu-Cl)_3Ru(MePh_2P)_3]$ | 100 | 0.63 | 97 |
| $[(Me_2PhP)_3Ru(\mu-Cl)_3Ru(Me_2PhP)_3]$ | 100 | 0.15 | 89 |

Example 7

To determine the effect of added phosphine compound to iridium catalyst, a run was carried out using $IrCl_3.xH_2O$ in the absence of phosphine or other catalyst modifier, under conditions employed in Example 1 above. In the reaction of morpholine with ethylene glycol at 20% conversion of the morpholine, hydroxylethylmorpholine was obtained at 10% selectivity and 1,2-bis(morpholino)ethane at 56% selectivity. The results obtained in the same reaction with 3 moles $PPh_3$ added per mole of $IrCl_3$ is reported in Table 1, Run No. 10. In the presence of $PPh_3$ higher overall conversion is obtained with the iridium catalyst and considerably higher selectivity (90%) to di-amination.

Attempts were made to aminate ethylene glycol with methylamine and with ammonia. Very little activity was seen as compared to the use of secondary amine as reactant. The little activity that was observed led to cyclic products (eq III) and only trace amounts of N-methylethanolamine.

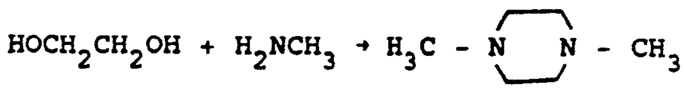

(III)

The use of ammonia was even less successful, with no amination products being observed. In fact, the addition of $NH_3$ to a solution in which morpholine amination of ethylene glycol was being catalyzed, was found to poison the catalysis of the amination reaction and to lead away from the desired production of ethanolamine and/or ethylenediamine.

While in the illustrative operating examples employing phosphine as a catalyst modifier, the ratio of P:Ru is up to about 3:1, the invention is not limited thereto. Good results have been obtained at P:Ru ratios up to 5:1, and while no special benefits are known to be obtained thereby, higher P:Ru ratios as up to about 10:1 may be employed.

**Claims**

1. In the amination of an alkanediol with a secondary amine in the presence of a platinum group catalyst, the method of selectively controlling the relative extent of (A) mono-amination and (B) diamination of said diol, characterised by effecting said amination at a temperature in the range of 50 to 200°C in the presence of catalyst comprising a ruthenium or iridium metal complex wherein said catalyst is provided by the introduction of (A) a compound or complex of ruthenium in chemical combination or in admixture with a catalyst modifier consisting essentially of an organic phosphine selected from the group consisting of $P(Ph)_3$, $P(p\text{-}C_6H_4F)_3$, $P(Ph)Me_2$, $P(p\text{-}tol)_3$, $P(C_6F_5)_3$, $P(OPh)_3$, DPPM, $PPh_2Me$, $P(i\text{-}Pr)_3$, DPPE and mixtures thereof to selectively favor mono-amination; or as (B) a catalyst selected from the group consisting of (1) a ruthenium compound or complex in the absence of such catalyst modifier, (2) an iridium compound or complex free of modifier or in admixture or in chemical combination with an organic phosphine modifier, and (3) a ruthenium compound in admixture or in chemical combination or admixture with a catalyst modifier consisting essentially of an organic phosphine selected from the group consisting of $PPh(C_6F_5)_2$, $P(o\text{-}C_6H_4NMe_2)$, $P\text{-}(o\text{-}tol)_3$ and mixture thereof, to selectively favor di-amination.

2. The method as defined in Claim 1 wherein said organic phosphine is a compound of the formula

$$PR_1R_2R_3$$

wherein $R_1$ and $R_2$ are each H or an alkyl or aryl hydrocarbyl group and $R_3$ is a hydrocarbyl group; each said hydrocarbyl group separately containing up to 12 carbon atoms.

3. The method as defined in Claim 2 favoring mono-amination, wherein said ruthenium catalyst is selected from the group consisting of: (1) $RuCl_2(PPh_3)_3$, $RuHCl(PPh_3)_3$; and (2) the following compounds in admixture with $PPh_3$:($Ru(NH_3)_6Cl_3$, $K_2((RuCl_5)_2O)$, $Ru(NO)Cl_3$, $K_2RuCl_5$, $K_2RuCl_6$, $(Ru(NH_3)_6)Cl_2$ and $K_2RuO_4$.

4. The method as defined in Claim 2 favoring mono-amination, wherein said ruthenium catalyst is a compound from the group consisting of: ($Ru(NH_3)_6Cl_3$, $K_2((RuCl_5)_2O$, $Ru(NO)Cl_3$, $K_2RuCl_5$, $K_2RuCl_6$, and $(Ru(NH_3)_6)Cl_2$, each admixed with about 3 moles of triphenylphosphine per mole of ruthenium compound.

5. The method as defined in Claim 1 wherein said catalyst favoring di-amination is selected from the group consisting of phosphine free $RuCl_3.xH_2O$ and $IrCl_3.xH_2O$.

6. The method as defined in Claim 2 wherein said catalyst favoring di-amination is $IrCl_3.xH_2O$ in chemical combination or in admixture with triphenyl phosphine.

7. The method as defined in Claim 2 wherein said amination is effected at a temperature in the range of 80 to 160°C.

8. The method as defined in Claim 2 wherein said amination is effected in the absence of added hydrogen and at autogenous pressure.

9. The method as defined in Claim 2 wherein said amination is carried out in the presence of added hydrogen at superatmospheric pressure.

10. The method as defined in Claim 2 wherein said diol is ethylene glycol.

11. The method as defined in Claim 10 wherein said secondary amine is morpholine.

12. The method as defined in Claim 10 wherein said secondary amine is dimethylamine or diethylamine.

13. The method as defined in Claim 10 wherein said secondary amine is di-isopropylamine.

14. The method as defined in Claim 8 wherein said secondary amine is N,N,N'-trimethylethylenediamine.

15. The method as defined in Claim 8 wherein said secondary amine is pyrrolidine.

16. The method as defined in Claim 2 wherein said diol is 1,2-propanediol or 1,3-propanediol.

17. The method as defined in Claim 16 wherein said secondary amine is dimethylamine.

18. The method as defined in Claim 2 wherein said catalyst is $RuCl_2(PPh_3)_3$, said secondary amine is

selected from the group consisting of dimethylamine, diethylamine and morpholine and said alkanediol is selected from the group consisting of ethylene glycol, and propanediol.

19. The method as defined in Claim 2 wherein said catalyst is $RuCl_3.xH_2O$ said secondary amine is selected from the group consisting of morpholine and dimethylamine and said alkanediol is selected from the group consisting of ethylene glycol and propanediol.

20. The method as defined in Claim 2 wherein said catalyst is $IrCl_3.xH_2O$ admixed with triphenyl phosphine in 1:3 molar ratio, said secondary amine is selected from the group consisting of dimethylamine and morpholine, and said alkanediol is selected from the group consisting of ethylene glycol and propanediol.

21. The method as defined in Claim 2 wherein said amination is carried out in liquid phase.

22. The method as defined in Claim 21 wherein said amination is carried out with the secondary amine dissolved in the diol at an amine concentration of about 1.8 molar and at a dissolved catalyst concentration of about one-hundredth that of the amine.

23. The method as defined in Claim 21 wherein the catalyst is dissolved in the liquid reaction medium.

24. The method as defined in Claim 2 wherein said catalyst is provided by introduction into the reaction medium of a compound or complex of ruthenium in chemical combination or in admixture with $P(p\text{-}C_6H_4F)_3$.

25. The method as defined in Claim 2 wherein said catalyst is provided by introduction into the reaction medium of a compound or complex of ruthenium in chemical combination or in admixture with $P(Ph)Me_2$.

26. The method as defined in Claim 2 wherein said catalyst is provided by introduction into the reaction medium of a compound or complex of ruthenium in chemical combination or in admixture with $P(p\text{-tol})_3$.

27. The method as defined in Claim 2 wherein said catalyst is provided by introduction into the reaction medium of a compound or complex of ruthenium in chemical combination or in admixture with bis-(diphenylphosphino)methane.

28. The method as defined in Claim 2 wherein said catalyst is provided by introduction into the reaction medium of a compound or complex of ruthenium in chemical combination or in admixture with $PPh_2Me$.

29. The method as defined in Claim 2 wherein said catalyst is provided by introduction into the reaction medium of a compound or complex of ruthenium in chemical combination or in admixture with $PPh(C_6F_5)_2$.

30. The method as defined in Claim 2 wherein said catalyst is provided by introduction into the reaction medium of a compound or complex of ruthenium in chemical combination or in admixture with $P(o\text{-tol})_3$.

**Patentansprüche**

1. Verfahren zur selektiven Kontrolle des relativen Ausmaßes von (A) der Mono-Aminierung und (B) Di-Aminierung in der Aminierung eines Alkandiols mit einem sekundären Amin in Gegenwart eines Katalysators der Platingruppe, dadurch gekennzeichnet, daß man die Aminierung bei einer Temperatur im Bereich von 50 bis 200°C in Gegenwart eines Katalysators durchführt, der einen Ruthenium- oder Iridiummetallkomplex enthält, wobei der Katalysator durch Einführung von (A) einer Verbindung oder eines Komplexes von Ruthenium in einer chemischen Kombination oder in Beimischung mit einem Katalysator-Modifizierungsmittel zur Verfügung gestellt wird, wobei das Modifizierungsmittel im wesentlichen aus einem organischen Phosphin besteht, das aus der Gruppe $P(PH)_3$, $P(p\text{-}C_6H_4F)_3$, $P(Ph)Me_2$, $P(p\text{-Tol})_3$, $P(C_6F_5)_3$, $P(OPh)_3$, DPPM, $PPh_2Me$, $P(i\text{-Pr})_3$ DPPE und Mischungen davon ausgewählt ist, um selektiv die Mono-Aminierung zu begünstigen; oder (B) als ein Katalysator, ausgewählt aus der Gruppe bestehend aus (1) einer Rutheniumverbindung oder einem Komplex ohne Katalysator-Modifizierungsmittels, (2) einer Iridiumverbindung oder einem Komplex frei vom Modifizierungsmittel oder in Beimischung oder in chemischer Kombination mit einem organischen Phosphin-Modifizierungsmittel und (3) einer Rutheniumverbindung in Beimischung oder in chemischer Kombination oder Beimischung mit einem Katalysator-Modifizierungsmittel, das im wesentlichen aus einem organischen Phosphin, ausgewählt aus der Gruppe $PPh(C_6F_5)_2$, $P(o\text{-}C_6H_4NMe_2)$, $P\text{-}(o\text{-Tol})_3$ und Mischungen davon besteht, um selektiv die Di-Aminierung zu begünstigen.

2. Verfahren nach Anspruch 1, worin das organische Phosphin eine Verbindung der Formel

$$PR_1R_2R_3$$

ist, worin $R_1$ und $R_2$ jeweils H oder eine Alkyl- oder eine Arylhydrocarbylgruppe sind und $R_3$ eine Hydrocarbylgruppe ist; jede Hydrocarbylgruppe enthält getrennt bis zu 12 Kohlenstoffatome.

3. Verfahren nach Anspruch 2, bei dem die Mono-Aminierung begünstigt wird, worin der Ruthenium-Katalysator ausgewählt ist aus der Gruppe bestehend aus: (1) $RuCl_2(PPh_3)_3$, $RuHCl(PPh_3)_3$; und (2) den folgenden Verbindungen in Beimischung mit $PPh_3$: $(Ru(NH_3)_6Cl_3$, $K_2((RuCl_5)_2O$, $Ru(NO)Cl_3$, $K_2RuCl_5$, $K_2RuCl_6$, $(Ru(NH_3)_6)Cl_2$ und $K_2RuO_4$.

4. Verfahren nach Anspruch 1, bei dem die Mono-Aminierung begünstigt wird, worin der Ruthenium-Katalysator eine Verbindung aus der Gruppe ist, die besteht aus: $(Ru(NH_3)_6Cl_3$, $K_2((RuCl_5)_2O$, $Ru(NO)Cl_3$, $K_2RuCl_5$, $K_2RuCl_6$ und $(Ru(NH_3)_6)Cl_2$, jeweils beigemischt mit etwa 3 Mol Triphenylphosphin pro Mol der Rutheniumverbindung.

5. Verfahren nach Anspruch 1, worin der Katalysator, der die Di-Aminierung begünstigt, ausgewählt ist aus der Gruppe bestehend aus phosphinfreiem $RuCl_3.xH_2O$ und $IrCl_3.xH_2O$.

6. Verfahren nach Anspruch 2, worin der Katalysator, der Di-Aminierung begünstigt, $IrCl_3.xH_2O$ in chemischer Verbindung oder Beimischung mit Triphenylphosphin ist.

7. Verfahren nach Anspruch 2, worin man die Aminierung bei einer Temperatur im Bereich von 80 bis 160°C durchführt.

8. Verfahren nach Anspruch 2, worin man die Aminierung in Abwesenheit von zugefügtem Wasserstoff und bei autogenem Druck durchführt.

9. Verfahren nach Anspruch 2, worin man die Aminierung in Gegenwart von zugefügtem Wasserstoff bei superatmosphärischem Druck durchführt.

10. Verfahren nach Anspruch 2, worin das Diol Ethylenglykol ist.

11. Verfahren nach Anspruch 10, worin das sekundäre Amin Morpholin ist.

12. Verfahren nach Anspruch 10, worin das sekundäre Amin Dimethylamin oder Diethylamin ist.

13. Verfahren nach Anspruch 10, worin das sekundäre Amin Diisopropylamin ist.

14. Verfahren nach Anspruch 8, worin das sekundäre Amin N,N,N'-Trimethylethylendiamin ist.

15. Verfahren nach Anspruch 8, worin das sekundäre Amin Pyrrolidin ist.

16. Verfahren nach Anspruch 2, worin das Diol 1,2-Propandiol oder 1,3-Propandiol ist.

17. Verfahren nach Anspruch 16, worin das sekundäre Amin Dimethylamin ist.

18. Verfahren nach Anspruch 2, worin der Katalysator $RuCl_2(PPh_3)_3$ ist, das sekundäre Amin aus der Gruppe bestehend aus Dimethylamin, Diethylamin und Morpholin ausgewählt ist und das Alkandiol aus der Gruppe bestehend aus Ethylenglykol und Propandiol ausgewählt ist.

19. Verfahren nach Anspruch 2, worin der Katalysator $RuCl_3.xH_2O$ ist, das sekundäre Amin aus der Gruppe bestehend aus Morpholin und Dimethylamin ausgewählt ist und das Alkandiol aus der Gruppe bestehend aus Ethylenglykol und Propandiol ausgewählt ist.

20. Verfahren nach Anspruch 2, worin der Katalysator $IrCl_3.xH_2O$ iin Beimischung mit Triphenylphosphin in einem 1:3 Molverhältnis ist, das sekundäre Amin aus der Gruppe bestehend aus Dimethylamin und Morpholin ausgewählt ist und das Alkandiol aus der Gruppe bestehend aus Ethylenglykol und Propandiol ausgewählt ist.

21. Verfahren nach Anspruch 2, worin man die Aminierung in der flüssigen Phase durchführt.

22. Verfahren nach Anspruch 21, worin man die Aminierung mit dem sekundären Amin, gelöst in dem Diol bei einer Aminkonzentration von etwa 1,8 molar und bei einer gelösten Katalysatorkonzentration von etwa einem hundertstel des Amins durchführt.

23. Verfahren nach Anspruch 21, worin der Katalysator im flüssigen Reaktionsmedium gelöst ist.

24. Verfahren nach Anspruch 2, worin der Katalysator durch Einführung einer Verbindung oder eines Komplexes von Ruthenium in chemischer Kombination oder in Beimischung mit $P(p\text{-}C_6H_4F)_3$ in das Reaktionsmedium zur Verfügung gestellt wird.

25. Verfahren nach Anspruch 2, worin der Katalysator durch Einführung einer Verbindung oder eines Komplexes von Ruthenium in chemischer Kombination oder in Beimischung mit $P(Ph)Me_2$ in das Reaktionsmedium zur Verfügung gestellt wird.

26. Verfahren nach Anspruch 2, worin der Katalysator durch Einführung einer Verbindung oder eines Komplexes von Ruthenium in chemischer Kombination oder in Beimischung mit $P(p\text{-}Tol)_3$ in das Reaktionsmedium zur Verfügung gestellt wird.

27. Verfahren nach Anspruch 2, worin der Katalysator durch Einführung einer Verbindung oder eines Komplexes von Ruthenium in chemischer Kombination oder in Beimischung mit bis-(Diphenylphosphino)methan in das Reaktionsmedium zur Verfügung gestellt wird.

28. Verfahren nach Anspruch 2, worin der Katalysator durch Einführung einer Verbindung oder eines Komplexes von Ruthenium in chemischer Kombination oder in Beimischung mit $PPh_2Me$ in das Reaktionsmedium zur Verfügung gestellt wird.

29. Verfahren nach Anspruch 2, worin der Katalysator durch Einführung einer Verbindung oder eines Komplexes von Ruthenium in chemischer Kombination oder in Beimischung mit $PPh(C_6F_5)_2$ in das Reaktionsmedium zur Verfügung gestellt wird.

30. Verfahren nach Anspruch 2, worin der Katalysator durch Einführung einer Verbindung oder eines Komplexes von Ruthenium in chemischer Kombination oder in Beimischung mit $P(o\text{-}Tol)_3$ in das Reaktionsmedium zur Verfügung gestellt wird.

**Revendications**

1. Dans l'amination d'un alkanediol avec une amine secondaire en présence d'un catalyseur du groupe platine, procédé de contrôle sélectif du degré relatif de (A) la mono-amination et (B) la diamination de ce diol, caractérisé en ce qu'on effectue cette amination à une température comprise dans la gamme de 50 à 200°C en présence d'un catalyseur comprenant un complexe de métal ruthénium ou iridium, ce catalyseur étant fourni par l'introduction de (A) un composé ou un complexe de ruthénium en combinaison chimique ou en mélange avec un modificateur de catalyseur consistant essentiellement en une phosphine organique choisie parmi le groupe consistant en $P(Ph)_3$, $P(p\text{-}C_6H_4F)_3$, $P(Ph)Me_2$, $P(p\text{-}tol)_3$, $P(C_6F_5)_3$, $P(OPh)_3$, DPPM, $PPh_2Me$, $P(i\text{-}Pr)_3$, DPPE et leurs mélanges, pour favoriser sélectivement une mono-amination; ou de (B) un

catalyseur choisi parmi le groupe consistant en (1) un composé ou complexe de ruthénium en absence de modificateur de catalyseur, (2) un composé ou un complexe d'iridium exempt de modificateur ou en mélange ou en combinaison chimique avec un modificateur à base de phosphine organique, et (3) un composé de ruthénium en mélange ou en combinaison chimique ou en mélange avec un modificateur de catalyseur consistant essentiellement en une phosphine organique choisie parmi le groupe consistant en $PPh(C_6F_5)_2$, $P(o\text{-}C_6H_4NMe_2)$, $P\text{-}(o\text{-}tol)_3$ et leurs mélanges, pour favoriser sélectivement une di-amination.

2. Procédé suivant la revendication 1, caractérisé en ce que la phosphine organique est un composé de formule:

$$PR_1R_2R_3$$

dans laquelle $R_1$ et $R_2$ sont chacun H ou un groupe hydrocarbyle aryle ou alkyle et $R_3$ est un groupe hydrocarbyle; chaque groupe hydrocarbyle contenant séparément jusqu'à 12 atomes de carbone.

3. Procédé suivant la revendication 2, favorisant la mono-amination, caractérisé en ce que le catalyseur au ruthénium est choisi parmi le groupe consistant en: (1) $RuCl_2(PPh_3)_3$, $RuHCl(PPh_3)_3$; et (2) les composés suivants en mélange avec $PPh_3$: $(Ru(NH_3)_6Cl_3$, $K_2((RuCl_5)_2O)$, $Ru(NO)Cl_3$, $K_2RuCl_5$, $K_2RuCl_6$, $(Ru(NH_3)_6)Cl_2$ et $K_2RuO_4$.

4. Procédé suivant la revendication 2, favorisant la mono-amination, caractérisé en ce que le catalyseur au ruthénium est un composé du groupe consistant en $(Ru(NH_3)_6Cl_3$, $K_2((RuCl_5)_2O$, $Ru(NO)Cl_3$, $K_2RuCl_5$, $K_2RuCl_6$ et $(Ru(NH_3)_6)Cl_2$, chacun étant mélangé avec environ 3 moles de triphénylphosphine par mole du composé de ruthénium.

5. Procédé suivant la revendication 1, caractérisé en ce que le catalyseur favorisant la di-amination est choisi parmi le groupe consistant en $RuCl_3.xH_2O$ et $IrCl_3.xH_2O$ exempts de phosphine.

6. Procédé suivant la revendication 2, caractérisé en ce que le catalyseur favorisant la di-amination est le $IrCl_3.xH_2O$ en combinaison chimique ou en mélange avec la triphényl phosphine.

7. Procédé suivant la revendication 2, caractérisé en ce que l'amination est effectuée à une température comprise dans la gamme de 80 à 160°C.

8. Procédé suivant la revendication 2, caractérisé en ce que l'amination est effectuée en absence d'hydrogène additionnel et à une pression autogène.

9. Procédé suivant la revendication 2, caractérisé en ce que l'amination est effectuée en présence d'hydrogène additionnel à une pression superatmosphérique.

10. Procédé suivant la revendication 2, caractérisé en ce que le diol est l'éthylène glycol.

11. Procédé suivant la revendication 10, caractérisé en ce que l'amine secondaire est la morpholine.

12. Procédé suivant la revendication 10, caractérisé en ce que l'amine secondaire est la diméthylamine ou la diéthylamine.

13. Procédé suivant la revendication 10, caractérisé en ce que l'amine secondaire est la di-isopropylamine.

14. Procédé suivant la revendication 8, caractérisé en ce que l'amine secondaire est la N,N,N'-triméthyléthylènediamine.

15. Procédé suivant la revendication 8, caractérisé en ce que l'amine secondaire est la pyrrolidine.

16. Procédé suivant la revendication 2, caractérisé en ce que le diol est le 1,2-propanediol ou le 1,3-propanediol.

17. Procédé suivant la revendication 16, caractérisé en ce que l'amine secondaire est la diméthylamine.

18. Procédé suivant la revendication 2, caractérisé en ce que le catalyseur est $RuCl_2(PPh_3)_3$, l'amine secondaire étant choisie parmi le groupe consistant en diméthylamine, diéthylamine et morpholine et l'alkanediol est choisi parmi le groupe consistant en éthylène glycol et propanediol.

19. Procédé suivant la revendication 2, caractérisé en ce que le catalyseur est $RuCl_3.xH_2O$, l'amine secondaire étant choisie parmi le groupe consistant en morpholine et diméthylamine et l'alkanediol parmi le groupe consistant en éthylène glycol et propanediol.

20. Procédé suivant la revendication 2, caractérisé en ce que le catalyseur est $IrCl_3.xH_2O$ en mélange avec la triphényl phosphine en un rapport molaire 1:3, l'amine secondaire étant choisie parmi le groupe consistant en diméthylamine et morpholine et l'alkanediol parmi le groupe consistant en éthylène glycol et propanediol.

21. Procédé suivant la revendication 2, caractérisé en ce que l'amination est effectuée en phase liquide.

22. Procédé suivant la revendication 21, caractérisé en ce que l'amination est effectuée avec l'amine secondaire en solution dans le diol, avec une concentration en amine environ 1,8 molaire et avec une concentration du catalyseur en solution égale à environ un centième de l'amine.

23. Procédé suivant la revendication 21, caractérisé en ce que le catalyseur est en solution dans un milieu réactionnel liquide.

24. Procédé suivant la revendication 2, caractérisé en ce que le catalyseur est fourni par introduction dans le milieu réactionnel d'un composé ou d'un complexe de ruthénium en combinaison chimique ou en mélange avec $P(p\text{-}C_6H_4F)_3$.

25. Procédé suivant la revendication 2, caractérisé en ce que le catalyseur est fourni par introduction dans le milieu réactionnel d'un composé ou d'un complexe de ruthénium en combinaison chimique ou en mélange avec $P(Ph)Me_2$.

26. Procédé suivant la revendication 2, caractérisé en ce que le catalyseur est fourni par introduction dans le milieu réactionnel d'un composé ou d'un complexe de ruthénium en combinaison chimique ou en mélange avec $P(p\text{-}tol)_3$.

27. Procédé suivant la revendication 2, caractérisé en ce que le catalyseur est fourni par introduction dans le milieu réactionnel d'un composé ou d'un complexe de ruthénium en combinaison chimique ou en mélange avec le bis-(diphénylphosphino)méthane.

28. Procédé suivant la revendication 2, caractérisé en ce que le catalyseur est fourni par introduction dans le milieu réactionnel d'un composé ou d'un complexe de ruthénium en combinaison chimique ou en mélange avec $PPh_2Me$.

29. Procédé suivant la revendication 2, caractérisé en ce que le catalyseur est fourni par introduction dans le milieu réactionnel d'un composé ou d'un complexe de ruthénium en combinaison chimique ou en mélange avec $PPh(C_6F_5)_2$.

30. Procédé suivant la revendication 2, caractérisé en ce que le catalyseur est fourni par introduction dans le milieu réactionnel d'un composé ou d'un complexe de ruthénium en combinaison chimique ou en mélange avec $P(o\text{-}tol)_3$.